(19) **Europäisches Patentamt** / **European Patent Office** / **Office européen des brevets**

(11) **EP 4 663 158 A1**

(12) **EUROPEAN PATENT APPLICATION**

(43) Date of publication:
17.12.2025 Bulletin 2025/51

(21) Application number: 25180396.1

(22) Date of filing: 03.06.2025

(51) International Patent Classification (IPC):
**A61C 13/00** (2006.01)

(52) Cooperative Patent Classification (CPC):
**A61C 13/0022**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 06.06.2024 JP 2024092649

(71) Applicant: **Shofu Inc.**
**Kyoto 605-0983 (JP)**

(72) Inventors:
• **ITO, Shuma**
**Kyoto, 605-0983 (JP)**
• **NONAKA, Kazumichi**
**Kyoto, 605-0983 (JP)**

(74) Representative: **Eisenführ Speiser**
**Patentanwälte Rechtsanwälte PartGmbB**
**Gollierstraße 4**
**80339 München (DE)**

(54) **ZIRCONIA PRE-SINTERED BODY FOR DENTAL CUTTING AND MACHINING MOLDED AT DIFFERENT PRESS PRESSURE AND MANUFACTURING METHOD THEREOF**

(57) To manufacture a dental zirconia pre-sintered body for cutting and machining with little warpage without limiting the powder composition to be stacked to specific coloring materials or stabilizers, and with no distortion after perfect sintering.

In the step of forming a second or more layers, a zirconia powder for layering used in a green compact constituting each layer is different from a zirconia powder for layering used in a green compact constituting adjacent layer in relative density to the perfect sintered body in the green compact pressed at a press pressure of 4.78 $kN/cm^2$, and each press pressure of the powder compacting in forming the green compact that constitutes each layer is adjusted so that the difference in relative density between the green compacts constituting respective layers is 2 or less, and in the step of pre-sintering, the multilayer zirconia green compact is pre-sintered at a temperature at which the processing coefficients of respective layers after pre-sintering are substantially equal.

**Description**

BACKGROUND OF THE INVENTION

Field of the Invention

The present invention relates to a zirconia pre-sintered body for dental cutting and machining molded at different press pressures for preparing a dental restoration using CAD/CAM, and a manufacturing method thereof.

Description of the Related Art

In recent years, techniques to prepare a prosthesis device by the cutting and machining which uses the dental CAD/CAM system has been spread rapidly and therefore it has been becoming possible to easily prepare a prosthesis device by cutting and machining a mill blanks which is made of ceramic materials such as a zirconia, an alumina and a lithium disilicate glass, or resin materials such as an acrylic resin and a hybrid resin.

In particular, zirconia has been clinically applied in various cases because of its high strength. On the other hand, since a zirconia which is fully (perfect) sintered zirconia (hereinafter, referred to as "zirconia sintered body") has a very high hardness, a zirconia which is calcined at a temperature lower than the perfect sintering temperature to adjust to a hardness that enables to cut and machine (zirconia pre-sintered body) has been used for machining, and it is often to obtain a zirconia sintered body by re-firing after cutting and machining.

On the other hand, as the method of further enhancing the aesthetic property of the oral cavity, a method for obtaining a zirconia pre-sintered body by stacking zirconia powders with different contents of metal element which is a coloring material and yttria which improves translucency.

However, when zirconia powders with different compositions are powder compacted at the same pressure and calcined, there is a problem that warpage occurs in the zirconia pre-sintered body due to the difference in relative density.

Japanese Unexamined Patent Application Publication No. 2020-147494 discloses a manufacturing method for a sintered body with little warpage and little deformation by adjusting the content of zirconia and stabilizer contained in each zirconia layer.

Japanese Patent No. 6393328 discloses a ceramic body sintered without distortion by promoting densification to adjust the sintering behavior of each layer by incorporating a dopant in a multilayer oxide ceramic body in which ceramic materials with different sintering behaviors are stacked.

SUMMARY OF THE INVENTION

Technical Problem

However, because the conventional sintered body and pre-sintered body are affected by the composition of the powder to be stacked, the composition of the coloring material and stabilizer are limited, therefore it is impossible to adequately reproduce tooth color.

Because a zirconia pre-sintered body with large warpage cannot be attached to the clamp of a cutting and machining machine, steps such as flat grinding to remove the warpage of the zirconia pre-sintered body are required, therefore the number of manufacturing steps significantly increases. Thus, there has been a need for a simple method to reduce the warpage of a zirconia pre-sintered body so that tooth color can be reproduced in greater detail without limiting the composition to specific coloring materials or stabilizers.

Solution to Problem

As a result of intensive study for manufacturing methods for a dental zirconia pre-sintered body for cutting and machining with no warpage, it has been found that it is effective for suppressing warpage in a dental zirconia pre-sintered body for cutting and machining to perform powder compacting for each layer during stacking and to adjust the press pressure. Furthermore, it has been found that pre-sintering at a temperature that matches the processing coefficient is effective in suppressing distortion after perfect sintering. The details of the present invention are described below.

The present invention provides a method of manufacturing a zirconia pre-sintered body for dental cutting and machining which has a multilayer structure in which two or more layers are stacked, comprising steps of:

forming a green compact that constitutes a first layer by filling a mold with a zirconia powder for stacking and by powder compacting,
forming a multilayer zirconia green compact by forming a second or more layers by filling a position adjacent the formed green compact in the mold with a zirconia powder for stacking and by powder compacting,

performing isostatic press process on multilayer zirconia green compact, and

pre-sintering the multilayer zirconia green compact performed with the isostatic press process, wherein

in the step of forming a second or more layers,

a zirconia powder for layering used in a green compact constituting each layer is different from a zirconia powder for layering used in a green compact constituting adjacent layer in relative density to the perfect sintered body in the green compact pressed at a press pressure of 4.78 kN/cm$^2$, and

each press pressure of the powder compacting in forming the green compact that constitutes each layer is adjusted so that the difference in relative density between the green compacts constituting respective layers is 2 or less, and

in the step of pre-sintering,

the multilayer zirconia green compact is pre-sintered at a temperature at which the processing coefficients of respective layers after pre-sintering are substantially equal.

Advantageous Effects of Invention

According to the present invention, it is possible to manufacture a dental zirconia pre-sintered body for cutting and machining with little warpage without limiting the powder composition to be stacked to specific coloring materials or stabilizers, and with no distortion after perfect sintering.

DETAILED DESCRIPTION OF THE PREFERRED EMBODIMENTS

In the present invention, in the step of forming a multilayer zirconia green compact, each press pressure of the powder compacting in forming the green compact that constitutes each layer may be adjusted so that the difference in relative density between the green compacts constituting each layer is 1 or less.

In the present invention, in the step of forming a multilayer zirconia green compact, each press pressure of the powder compacting in forming the green compact that constitutes each layer may be adjusted so that the difference in relative density between the green compacts constituting each layer is 0.5 or less.

In the present invention, the differences between the press pressure of powder compacting in forming a green compact constituting each layer and the press pressure of powder compacting in forming a green compact constituting the other layers may be 0.1 kN/cm$^2$ or more.

In the present invention, when the relative densities of the green compacts obtained by pressing the zirconia powder for layering used in the green compacts constituting the first to $n^{th}$ layers at a pressure of 4.78 kN/cm$^2$ are defined as $D_1$ to $D_n$, respectively, the relationship $D_1 < D_2 < ... < D_{n-1} < D_n$ may be satisfied.

In the present invention, when the press pressures in forming the green compacts constituting the first layer to $n^{th}$ layer are defined as $P_1$ to $P_n$, respectively, the relationship the relationship $P_n < P_{n-1} < ... P_2 < P_1$ may be satisfied.

In the present invention, the zirconia powder for layering may contain a sintering aid.

In the present invention, the zirconia powder for layering may contain 80 % by weight of zirconia in terms of weight.

In the present invention, a holding time of the press pressure may be 1 second or more in forming the green compact constituting each layer.

In the present invention, in the step of performing isostatic press process, cold isostatic pressing (CIP) and/or warm isostatic pressing (WIP) may be performed.

In the present invention, the pressure of the cold isostatic pressing (CIP) and/or warm isostatic pressing (WIP) may be 100 MPa or more.

In the present invention, the pressure holding time of maximum pressure in cold isostatic pressing (CIP) and/or warm isostatic pressing (WIP) may be 30 seconds or more.

In the present invention, the multilayer zirconia green compact performed with the isostatic press process may be pre-sintered at 800 to 1200 °C.

The present invention provides a dental zirconia pre-sintered body for cutting and machining manufactured by the manufacturing method of the dental zirconia pre-sintered body for cutting and machining of the present invention.

Hereinafter, specific embodiments of the present invention will be described in detail.

The zirconia powder for layering in the present invention is used to manufacture a dental zirconia pre-sintered body for cutting and machining.

The zirconia powder for layering in the present invention may contain 80 % by weight or more, 85 % by weight or more, 90 % by weight or more, or 95 % by weight or more of zirconia in terms of weight. When the zirconia content is less than 80 % by weight, there is a tendency that the translucency and strength decrease. The zirconia powder for layering in the present invention may contain less than 80 % by weight of zirconia in terms of weight. Furthermore, in the present invention, only zirconia powder containing 80 % by weight or more of zirconia may be used as the zirconia powder for layering in terms of weight.

The zirconia powder for layering in the present invention may contain a stabilizer that suppresses the phase transition of zirconia. The stabilizer is not particularly limited, but examples include yttria, ceria, calcia, magnesia, and the like. In the present invention, these stabilizers may be used alone or in combination. The zirconia powder for layering in the present invention may contain only one or more stabilizers selected from the group consisting of yttria, ceria, calcia, and magnesia. In the present invention, the total amount of the stabilizer in the zirconia powder for layering may be, for example, 0.1 mol % or more and 13.0 mol % or less. In particular, when yttria is used as the stabilizer, the content is preferably 1.0 mol % or more and 10.0 mol % or less, with the remainder being zirconia. In the present invention, a zirconia powder for layering that does not contain the stabilizer may be used.

The zirconia powder for layering in the present invention may contain a coloring material. Specific examples thereof include iron oxide for imparting yellow color and erbium for imparting red color. In addition, there is no problem at all even if a coloring material containing an element such as cobalt, manganese, and chromium for adjusting color in addition to these coloring materials is used together. Other examples include various metal elements such as nickel, praseodymium, neodymium, gadolinium, and terbium, and by including a coloring material in zirconia powder, it is possible to reproduce a natural tooth in greater detail. In the present invention these coloring materials may be used alone or in combination of two or more. The zirconia powder for layering in the present invention may contain only one or more selected from the group consisting of iron oxide, erbium, cobalt, manganese, chromium, nickel, praseodymium, neodymium, gadolinium, and terbium as the coloring material. In the present invention, the total amount of the coloring material in the zirconia powder for layering may be, for example, 0.0001 % by weight or more and 2.0 % by weight or less. In the present invention, the zirconia powder for layering that does not contain the coloring material may be used.

The zirconia powder for layering in the present invention may contain a sintering aid, and examples include alumina and silica. In the present invention, these sintering aids may be used alone or in combination. The zirconia powder for layering in the present invention may contain only one or more stabilizers selected from the group consisting of alumina and silica. The content of the sintering aid contained in the zirconia powder for layering may be determined based on the sintering temperature and yhe property of the intended sintered body. In the present invention, the total amount of the sintering aid in the zirconia powder for layering may be, for example, 0 % by weight or more and 1.0 % by weight or less. In particular, in the case of alumina, the content is preferably 0 % by weight or more and 0.2 % by weight or less, more preferably 0 % by weight or more and 0.1 % by weight or less, and further preferably 0 % by weight or more and 0.05 % by weight or less. The higher the content of the sintering aid, the lower the sintering temperature of the zirconia sintered body, but the lower the translucency, so it is necessary to add an appropriate amount. In the present invention, a zirconia powder for layering that does not contain the sintering aid may be used.

It is preferable that a primary particle diameter of the zirconia powder for layering of the present invention is within a range of 1 to 500 nm. When the primary particle diameter is less than 1 nm, there is a tendency that it is difficult to impart sufficient strength, although the translucency of the zirconia sintered body is improved. On the other hand, when the primary particle diameter is more than 500 nm, there is a tendency that it is difficult to impart sufficient strength to the zirconia sintered body. The zirconia powder for layering in the present invention may consist of only the zirconia powder having a primary particle diameter within a range of 1 to 500 nm. In the present invention, the primary particle diameter of the zirconia powder for layering may not be within a range of 1 to 500 nm.

It is preferable that the zirconia powder for layering in the present invention is a powder that is granulated (hereinafter referred to as granulated powder) for the purpose of improving operability and moldability. The granulated powder is granulated by a granulation method such as a spray drying method, and the particle diameter after granulation is preferably 20 μm or more and 100 μm or less.

The zirconia powder for layering in the present invention may be a mixture of multiple types of the zirconia powders. By mixing zirconia powders with different contents of yttria and/or a coloring material, it is possible to express translucency and color tone in more detail, thereby improving the reproducibility of a natural tooth.

The zirconia powder for layering in the present invention is classified into at least two types of the zirconia powders for layering. In the present invention, the zirconia powder for layering may be classified based solely on the relative density to the perfect sintered body in the green compact described below. That is, even if zirconia powders have different contents of the stabilizer, the coloring material and the sintering aid, they can be considered the same type of the zirconia powder for layering when they have the same relative density.

The multilayer zirconia green compact used in manufacturing the dental zirconia pre-sintered body for cutting and machining in the present invention is prepared by repeatedly performing the powder compacting of the zirconia powder for layering.

The shape of the multilayer zirconia green compact in the present invention is preferably a disk shape or a block shape, but is not limited to these shapes and may be a shape that is convenient for processing and depends on the desired processed product such as a semi-disk shape that approximates the contour based on the shape of the human upper jaw, an oval shape, and a polygon shape.

The multilayer zirconia green compact of the present invention consists of two or more types of the zirconia powders for layering which are difference in the relative density to perfect sintered body in green compact pressed at a press pressure of 4.78 kN/cm$^2$ each other. In the present invention, the "relative density to perfect sintered body in green compact" is determined based on the density of a zirconia sintered body obtained by perfect sintering a zirconia green compact. The density is calculated from the apparent volume and the mass. For example, by pressing a zirconia powder for layering at a surface pressure of 4.78 kN/cm$^2$ and determining the density of the green compact after pressing, the relative density to perfect sintered body in green compact at a pressure of 4.78 kN/cm$^2$ can be calculated using the following formula.

Relative density to perfect sintered body in green compact (%) = {(density of zirconia green compact) / (density of zirconia sintered body)} x 100          [Formula 1]

In the present invention, "two or more types of the zirconia powders for layering which are difference in the relative density to perfect sintered body in green compact" means powders in which the difference in the relative density to perfect sintered body in green compact obtained by powder compacting at a surface pressure of 4.78 kN/cm$^2$ is 0.5 or more. In other words, in the present invention, "the relative density is different between the zirconia powders for layering" means that the difference in the relative density in the green compact between the zirconia powders for layering is 0.5 or more.

The zirconia powder for layering used in the multilayer zirconia green compact of the present invention can be arbitrarily detemined as long as it can constisute two or more types of the zirconia powders for layering which are difference in the relative density to perfect sintered body in green compact. For example, when it is defined that the number of layers in the multilayer zirconia green compact to be molded is n and the respective layers are numbered from one side of the stacking direction as the first layer, the second layer, ... the n$^{th}$ layer, n types of the zirconia powders for layering which are difference in the relative density each other (the fist type zirconia powder for layering, the second type zirconia powder for layering, ... the n$^{th}$ type zirconia powder for layering) may be prepared. In this case, the respective zirconia powders for layering may be stacked, such as the first type of zirconia powder for layering is in the first layer, the second type of zirconia powder for layering is in the second layer, ... the n$^{th}$ type of zirconia powder for layering is in the n$^{th}$ layer.

In the zirconia powder for layering used in the multilayer zirconia green compact of the present invention, as long as it can constisute two or more types of the zirconia powders for layering which are difference in the relative density to perfect sintered body in green compact, the number of types of the zirconia powders for layering to be used may be fewer than the number of layers of the multilayer zirconia green compact to be molded. For example, a multilayer zirconia green compact having five layers may be obtained by stacking three consecutive layers of three kinds of the first type of the zirconia powder for layering that have different compositions but have the same relative density or a difference less than 0.5 in the relative density, one layer of the second type of the zirconia powder for layering that has a difference in the relative density from the first type of the zirconia powder for layering, and one layer of the third type of the zirconia powder for layering that has a difference in the relative density from the first type zirconia powder for layering and the second type zirconia powder for layering, and then powder compacting.

In the multilayer zirconia green compact in the present invention, the powder compacting is performed on each time the zirconia powder for layering is filled into each layer. In the case of molding a multilayer zirconia green compact having an n-layer structure, for example in the case of n = 3, the first type of the zirconia powder for layering is filled into a mold and powder compacted at press pressure (1), the second type of the zirconia powder for layering is filled into the mold at a position continuous thereto and powder compacted at press pressure (2), and the n$^{th}$ type of the zirconia powder for layering is filled into the mold at a position continuous thereto and powder compacted at press pressure (n) to form a multilayer zirconia green compact. In forming the second or more layers, a zirconia powder for layering that is newly filled and has not been powder compacted may be powder compacted together with the zirconia powder for layering that has already been powder compacted to form a green compact.

The multilayer zirconia green compact of the present invention consists of at least two or more types of the zirconia powders for layering which are difference in the relative density to the perfect sintered body in the green compact pressed at a press pressure of 4.78 kN/cm$^2$ each other. Therefore, the relative densities of the green compacts in the respective layers are adjusted by powder compacting the respective layers with different press pressures. Specifically, for example in the case of two layers, the relative density is measured when the first type of the zirconia powder for layering is powder compacted at press pressure (1). The second type of the zirconia powder for layering is filled into the mold at a position continuous thereto and powder compacted at press pressure (2). At this time, the second type of the zirconia powder for layering is powder compacted at press pressure (2) such that the difference in the relative density between the green compact of the first layer powder compacted at the press pressure (1) is 2 or less. The difference in the relative density in the green compact in the case of powder compacting the first type of the zirconia powder for layering and the second type of the zirconia powder for layering is more preferably 1 or less, and further preferably 0.5 or less. When the difference in the relative density in the green compact is greater than 2, the warpage in the pre-sintered body after calcination becomes large.

In the present invention, in the case of stacking n types of the zirconia powders for layering which are difference in the relative density each other, the press pressure (1) to press pressure (n) of the powder compacting in forming the green compacts that constitute the respective layers are adjusted so that the difference in the relative density between the green compacts constituting the respective layers is 2 or less. In other words, each of the press pressure of the powder compacting in forming the green compact that constitutes each layer is adjusted so that the maximum difference in the relative density between two green compacts selected from the green compacts which constitute r the espective layers of the multilayer zirconia green compact is 2 or less. In other words, each of the press pressure of the powder compacting in forming the green compact that constitutes each layer is adjusted so that the difference between the maximum relative density and the minimum relative density of the green compacts that constitutes the respective layers of the multilayer zirconia green compact is 2 or less. For each of the press pressure of the powder compacting in forming the green compact constituting each layer, it is preferable that the difference in relative density between the green compacts constituting the respective layers is 1 or less, more preferably 0.5 or less.

The respective differences in the surface pressure between the press pressure (1), the press pressure (2) and the press pressure (n) are preferably 0.1 kN/cm$^2$ or more, more preferably 0.3 N/cm$^2$ or more, and most preferably 0.5 kN/cm$^2$ or more. In other words, the respective differences in the surface pressure between the press pressure of the powder compacting in forming a green compact constituting each layer and the press pressure of the powder compacting in forming a green compact constituting the others layer are preferably 0.1 kN/cm$^2$ or more, more preferably 0.3 kN/cm$^2$ or more and most preferably 0.5 kN/cm$^2$ or more. When one or more of the respective differences in the surface pressure between the press pressure (1), the press pressure (2), and the press pressure (n) are less than 0.1 kN/cm$^2$, there is a tendency that it is difficult to adjust the relative density of each layer. Furthermore, because the press pressure in the final powder compacting is less than 0.1 kN/cm$^2$, there is a tendency that the green compact becomes brittle to reduce workability. In the present invention, the differences in the surface pressure between the press pressure of powder compacting in forming a green compact constituting each layer and the press pressure of powder compacting in forming a green compact constituting the other layers may be not more than 0.1 kN/cm$^2$.

When the relative density of the green compact of the first type fo the zirconia powder for layering at a press pressure of 4.78 kN/cm$^2$ is defined $D_1$, the relative density of the green compact of the second type of the zirconia powder for layering at a press pressure of 4.78 kN/cm$^2$ is defined $D_2$, and the relative density of the green compact of the $n^{th}$ type of the zirconia powder for layering at a press pressure of 4.78 kN/cm$^2$ is defined Dn, it is preferable to satisfy the relationship $D_1 < D_2 < D_n$. In other words, when the relative densities of the green compacts obtained by pressing the zirconia powders for layering used in the green compacts constituting the first to $n^{th}$ layers at a pressure of 4.78 kN/cm$^2$ are defined $D_1$ to $D_n$, respectively, it is preferable to satisfy the relationship $D_1 < D_2 < ... < D_{n-1} < D_n$. By satisfying this relationship, in forming the second or more layers, in the case of powder compacting a zirconia powder for layering that is newly filled and has not been powder compacted together with the zirconia powder for layering that has already been powder compacted to form the green compact, in adjusting the relative density of each layer in the press process, the press pressure required to adjust the relative density of each layer can be made weaker than the press pressure required to adjust the relative density of the adjacent layer that already forms the green compact. Therefore, it is possible to stack layers without affecting the relative density of the adjacent layer that already forms the green compact.

It is preferable that the press pressure (1), the press pressure (2), and the press pressure (n) satisfy the relationship: press pressure (n) < press pressure (2) < press pressure (1). In other words, when the press pressures in forming the green compacts constituting the first layer to $n^{th}$ layer are defined as $P_1$ to $P_n$, respectively, it is preferable to satisfy the relationship $P_n < P_{n-1} < ... < P_2 < P_1$. By satisfying this relationship, in forming the second or more layers, in the case of powder compacting a zirconia powder for layering that is newly filled and has not been powder compacted together with the zirconia powder for layering that has already been powder compacted to form the green compact, the press pressure applied to each layer can be made weaker than the press pressure applied to the adjacent layer that already forms the green compact. Therefore, it is possible to stack layers without affecting the relative density of the adjacent layer that already forms the green compact.

In the present invention, it is preferable that a holding time of the press pressure in forming the green compact constituting each layer is 1 second or more. The holding time of the press pressure is more preferably 30 seconds or more, and further preferably 60 seconds or more. When the holding time of the press pressure of the maximum pressure is less than 1 second, there is a case where the zirconia powder for layering is not pressed uniformly and it is difficult to adjust the relative density of each layer. There is a tendency that the longer the holding time of the press pressure, the easier it becomes to adjust the relative density of each layer. However, because the molding time increases and productivity decreases, it is necessary to select the holding time of the press pressure appropriately. In the present invention, the holding time of the press pressure in forming the green compact constituting each layer may be less than 1 second.

In the present invention, the step of powder compacting the $n^{th}$ type of the zirconia powder for layering at press pressure (n) is the final powder compacting step. The holding time of the press pressure (n) is preferably 30 seconds or more. The holding time of the press pressure (n) is more preferably 60 seconds or more. In the present invention, the holding time of the press pressure (n) may be less than 30 seconds.

In the multilayer zirconia green compact in the present invention, an isostatic pressing process is performed after the final powder compacting step. In the multilayer zirconia green compact after the final powder compacting step in which this isostatic press process is to be performed, the differences in the relative density between the green compacts constituting the respective layers are adjusted to be 2 or less. Here, the sintering behavior (shrinkage rate and shrinkage start temperature) until perfect sintering differs depending on, for example, the composition of the zirconia powder for layering. Then, when the multilayer zirconia green compact is pre-sintered, the pre-sintering causes sintering (shrinkage) to progress to a certain extent. Therefore, even if the differences in the relative density between the green compacts constituting the respective layers is adjusted to be 2 or less, in the case of performing pre-sintering without an isostatic pressing process, the progress of sintering (shrinkage) at the pre-sintering temperature causes a difference in the relative density between the respective layers of the pre-sintered body, which causes distortion after perfect sintering. In other words, when a multilayer zirconia green compact manufactured by stacking and molding a plurality of zirconia powders for layering with different sintering (shrinkage) behaviors is pre-sintered without performing the isostatic pressing process, there is a case where a temperature at which the processing coefficients of the respective layers in the pre-sintered body are substantially equal (pre-sintering temperature) is not exist. In this regard, in the multilayer zirconia green compact of the present invention, an isostatic press process is performed after the final powder compacting step. Therefore, due to the difference in the shrinkage rate betwenn the zirconia powders for layering against the pressure of the isostatic press process, differences in the relative density between the respective layers are already caused in the multilayer zirconia green compact after the isostatic pressing process, before pre-sintering. Thus, it is possible to definitely exist a temperature at which the difference in the relative density between the respective layers caused by the isostatic pressing process is cancelled out by the difference in sintering behavior at the pre-sintering temperature, that is a temperature at which the processing coefficients of the respective layers in the pre-sintered body are substantially equal.

In the present invention, it is preferable that the isostatic pressing process is cold isostatic pressing (CIP) and/or warm isostatic pressing (WIP). By performing these isostatic pressing processes, it is possible to definitely exist a temperature at which the processing coefficients of the respective layers after pre-sintering are substantially equal and to improve the properties (translucency and strength) of the zirconia sintered body after perfect sintering. The pressure of the cold isostatic pressing (CIP) and/or warm isostatic pressing (WIP) may be 100 MPa or more, is preferably 100 to 300 MPa, more preferably 150 to 250 MPa, and further preferably 175 to 225 MPa. When the pressure of the isostatic pressing is lower than 100 MPa, there is a case where the difference in the relative density between the layers disappear, and it is difficult to exist a temperature at which the processing coefficients of the respective layers after pre-sintering are substantially equal. In addition, there is a case where the properties (translucency and strength) of the zirconia sintered body after perfect sintering decrease. When the pressure of the isostatic press is higher than 300 MPa, there is a tendency that the manufacture time is running out, and the running costs increase, therefore productivity decreases. In the present invention, the pressure of the cold isostatic pressing (CIP) and/or warm isostatic pressing (WIP) may be less than 100 MPa.

In the present invention, the holding time of the press presure of the maximum pressure in the isostatic press process, particularly the holding time of the press presure of the maximum pressure in cold isostatic pressing (CIP) and/or warm isostatic pressing (WIP), is preferably 30 seconds or more, more preferably 60 seconds or more. When the holding time of the press presure of the maximum pressure is less than 30 seconds, there is a tendency that density unevenness occurs in the green compact. In the present invention, the holding time of the press presure of the maximum pressure in the isostatic press process may be less than 60 seconds.

In the present invention, the dental zirconia pre-sintered body for cutting and machining is a multilayer zirconia green compact that has been fired (pre-sintered) to a state where the zirconia crystal particles are not perfectly sintered. After installing in a dental milling machine, an arbitrary prosthesis device is cut and machined from a dental zirconia pre-sintered body for cutting and machining, and a dental zirconia sintered body for cutting and machining is prepared by perfect sintering so that it can be used in the oral cavity. Therefore, the density of the dental zirconia sintered body for cutting and machining is higher than that of the dental zirconia pre-sintered body for cutting and machining. The dental zirconia pre-sintered body for cutting and machining includes pores and therefore is a porous body. The value calculated by the apparent volume and the mass is called as the density of the dental zirconia pre-sintered body for cutting and machining. The dinsity of the sintered body of the dental zirconia sintered body for cutting and machining is calculated by the dimensional measurement and the mass measurement. The "relative density of dental zirconia pre-sintered body for cutting and machining" is calculated by following formula 2.

Relative density of dental zirconia pre-sintered body for cutting and machining (%) = {(density of dental zirconia pre-sintered body for cutting and machining) / (density of zirconia sintered body for dental cutting)} × 100          [Formula 2]

The relative density of the dental zirconia pre-sintered body for cutting and machining of the present invention is preferably 20 to 80 %, and more preferably 40 to 60 % for cutting with a dental milling machine. When the relative density is less than 20 %, there is a tendency that the Vickers hardness and/or bending strength become too low and therefore chipping and breakage easily occur in the cutting and machining. When the relative density is more than 80 %, there is a tendency that the Vickers hardness and/or bending strength become too high and therefore a milling bar of a milling machine is heavily consumed to raise a running cost.

The dental zirconia pre-sintered body for cutting and machining in the present invention is pre-sintered at a temperature such that the processing coefficients of the respective layers after pre-sintering are substantially equal. For example, in the case of defining the length, the width, and the height of the rectangular parallelepiped pre-sintered body of each layer as $X_{PS}$, $Y_{PS}$, and $Z_{PS}$, respectively, and the length, the width, and the height of each layer after perfect sintering as $X_S$, $Y_S$, and $Z_S$, respectively, the "processing coefficient" is a value that can be calculated from the following formula 3. In addition, in the present invention, "the processing coefficients of the respective layers after pre-sintering are substantially equal" means that the processing coefficients of the respective layers determined based on after pre-sintering and after perfect sintering are close to each other. The multilayer zirconia green compact of the present invention is powder compacted by matching the relative densities of the respective layers. Therefore, if the processing coefficients of the respective layers after pre-sintering and after perfect sintering are close to each other, it is possible to obtain a dental zirconia pre-sintered body for cutting and machining with little warpage and no distortion and no deformation after perfect sintering.

[Formla 3]

$$\text{Processing coefficient} = ((X_{PS}/X_S) + (Y_{PS}/Y_S) + (Z_{PS}/Z_S))\, /3$$

[Maximum difference in processing coefficients]

The processing coefficient of each layer after pre-sintering of the dental zirconia pre-sintered body for cutting and machining in the present invention may be determined based on the difference between the layer with the highest processing coefficient and the layer with the lowest processing coefficient (the maximum difference in the processing coefficient between respective layers), and is preferably 0.003 or less. Furthermore, when the difference in the processing coefficient between the respective layers is 0.003 or less, it may be considered that the processing coefficients of respective layers are substantially equal. When the difference in the processing coefficient between respective layers is 0.003 or less, distortion and deformation are less likely to occur after perfect sintering, and therefore fitting property of the prosthesis device is improved. The difference in the processing coefficient is more preferably 0.002 or less, and further preferably 0.001 or less.

The calcination temperature of the dental zirconia pre-sintered body for cutting and machining of the present invention is preferably 800 to 1200 °C. When the calcination temperature is less than 800 °C, there is a tendency that because Vickers hardness and/ or bending strength become too low and therefore chipping and breakage easily occur in the cutting and machining. On the other hand, when the calcination temperature is more than 1200 °C, there is a tendency that because Vickers hardness and/ or bending strength become too high and therefore a milling bar of a milling machine is heavily consumed to raise a running cost.

[Warpage amount of dental zirconia pre-sintered body for cutting and machining]

The amount of warpage of the dental zirconia pre-sintered body for cutting and machining in the present invention is a value calculated by the following formula 4, in the case of that the first layer surface of a dental zirconia pre-sintered body for cutting and machining manufactured into a disk shape having a diameter of 98 mm ($\pm$ 1.0 mm) and a thickness of 18 mm ($\pm$ 1.0 mm) is placed facing downward relative to a horizontally maintained standard plane, and the length from the standard plane measured at the center of the disk with a dial gauge is defined as L1, and the disk is inverted with the final layer surface facing downward, and the length from the standard plane measured at the center of the disk with the dial gauge is defined as L2. The amount of warpage of the dental zirconia pre-sintered body for cutting and machining is preferably 0.5 mm or less, more preferably 0.3 mm or less, and further preferably 0.05 mm or less. Although the dental zirconia pre-sintered body for cutting and machining preferably has no warpage (warpage of 0 mm), it may have warpage that cannot be measured with a gauge (warpage of 0 mm or more).

[Formula 4]

$$\text{Warpage amount (mm)} = |L1 - L2|$$

In this way, by the manufacturing method of the present invention, it is possible to obtain a dental zirconia pre-sintered body for cutting and machining with little warpage. The obtained dental zirconia pre-sintered body for cutting and machining is preferably cut, milled, and surface polished to the desired size as needed.

The kind of a prosthesis device prepared by cutting and machining the dental zirconia pre-sintered body for cutting and machining according to the present invention is not limited particularly, and there is no problem at all even if the prosthesis device is any of an inlay, a laminate, a crown, a bridge and the like. Therefore, a shape of a multilayer zirconia green compact which is cut and machined for preparing a prosthesis device is not limited particularly, and any zirconia mill blank for dental cutting and machining can be used even if the dental zirconia pre-sintered body for cutting and machining has any shape such as a block shape corresponding to an inlay, a laminate, a crown and the like and a disk shape corresponding to a bridge.

By perfect sintering the dental zirconia pre-sintered body for cutting and machining of the present invention, it is possible to obtain a dental zirconia sintered body for cutting and machining without distortion.

The method for sintering the dental zirconia pre-sintered body for cutting and machining to prepare a sintered body in a sintered state is not particularly limited, but a simple and preferable method is to firing at normal pressure. The firing temperature is not particularly limited, but is preferably 1400 to 1600 °C, particularly preferably 1450 to 1550 °C. The holding time at the maximum firing temperature is not particularly limited, but is preferably minute to 12 hours, and more preferably 10 minutes to 4 hours. The temperature increase rate is not particularly limited, but is preferably 1 to 400 °C/minute, and more preferably 1 to 100 °C/min.

[Example]

Hereinafter, example of the present invention are specifically described. However, the present invention is not intended to be limited to these Examples.

[Relative density of green compact of zirconia powder for layering pressed at a pressure of 4.78 kN/cm2 to that of the fully sintered body of the green compact]

Each of the zirconia powders Zpex ($ZrO_2$: 86 wt%), Zpex4 ($ZrO_2$: 85 wt%), and Zpex Smile ($ZrO_2$: 83 wt%) manufactured by Tosoh Corporation in 17 g was individually filled into a mold having $\Phi$20 mm and height 56 mm, and powder compacted at a surface pressure of 4.78 kN/cm$^2$ to obtain a green compact. The density of the obtained green compact was measured using the following formula (5). The green compact after measurement was subjected to CIP treatment by pressing at a pressure of 200 MPa for 60 seconds using a cold isostatic press machine. The green compact after the CIP treatment was pre-sintered for 30 minutes at 1000 °C using a dental laboratory porcelain furnace (AUSTROMAT 674i, manufactured by DEKEMA Dental-Keramikofen GmbH) according to the manual, to obtain a dental zirconia pre-sintered body for cutting and machining. The zirconia pre-sintered body was then fired at 1450 °C for 2 hours using a dental laboratory porcelain firing furnace (AUSTROMAT 674i, manufactured by DEKEMA Dental-Keramikofen GmbH) according to the manual to obtain a zirconia sintered body. The density of the obtained zirconia sintered body was measured using the following formula (5). Mass measurements were performed using an electronic balance (XP-204, manufactured by Mettler Toledo) according to the manual. The relative density of the green compact was calculated using the density values of the green compact and the zirconia sintered body according to formula 1. The calculated relative densities for each zirconia powder for layering are shown in Table 1. In the zirconia powders for layering used in the present examples, relative densities to the perfect sintered body in the green compact at a press pressure of 4.78 kN/cm$^2$ differed by 0.5 or more each other .

[Formula 5]

$$\text{Density} = (\text{Diameter (mm)} \times \text{Hight (mm)}) / (\text{Mass (g)})$$

[Table 1]

| Zirconia powder | Zpex | Zpex4 | Zpex Smile |
|---|---|---|---|
| Relative density of green compact after pressing at 4.78kN/cm2 | 46.95 | 48.52 | 49.13 |

[Relative density of green compact constituting each layer]

Each of the zirconia powders in 17 g was individually filled into a mold having $\Phi 20$ mm and height 56 mm, and powder compacted at a surface pressure of P kN/cm$^2$ to obtain a green compact. The density of the obtained green compact was measured using the formula 5 (the surface pressure P is the powder compacting pressure applied to each layer in powder compacting the multilayer zirconia green compact in each example and comparative example). The green compact after measurement was subjected to CIP treatment by pressing at a pressure of 200 MPa for 60 seconds using a cold isostatic press machine (Comparative Example 3 was not subjected to CIP treatment). The green compact after the CIP treatment was pre-sintered for 30 minutes at 1000 °C using a dental laboratory porcelain furnace (AUSTROMAT 674i, manufactured by DEKEMA Dental-Keramikofen GmbH) according to the manual, to obtain a dental zirconia pre-sintered body for cutting and machining. The zirconia pre-sintered body was then fired at 1450 °C for 2 hours using a dental laboratory porcelain firing furnace (AUSTROMAT 674i, manufactured by DEKEMA Dental-Keramikofen GmbH) according to the manual to obtain a zirconia sintered body. The density of the obtained zirconia sintered body was measured using the following formula (5). Mass measurements were performed using an electronic balance (XP-204, manufactured by Mettler Toledo) according to the manual. The relative density of the green compact was calculated from the density of the green compact and the density of the zirconia sintered body using the formula 1.

[Specifying pre-sintering temperature so that processing coefficients of respective layers after pre-sintering are substantially equal]

Each of the zirconia powders Zpex, Zpex 4, and Zpex Smile manufactured by Tosoh Corporation in 480 g was individually filled into a $\Phi 100$ mm mold and powder compacted at a surface pressure of P kN/cm$^2$ (the surface pressure P was the powder compacting pressure applied to each layer in powder compacting the multilayer zirconia green compact in each example and comparative example). The green compact after powder compacting was subjected to CIP treatment by pressing at a pressure of 200 MPa for 60 seconds using a cold isostatic press machine. The multilayer zirconia green compact after the CIP treatment was pre-sintered for 30 minutes at temperature T (T is the temperature from 900 °C to 1100°C in 25 °C increments) using a dental laboratory porcelain furnace (AUSTROMAT 674i, manufactured by DEKEMA Dental-Keramikofen GmbH) according to the manual, to obtain a dental zirconia pre-sintered body for cutting and machining. A rectangular parallelepiped in which the dimensions of the length, the width, and the height is approximately 18 mm x 18 mm x 19 mm was cut out from the obtained zirconia pre-sintered body using a dental milling machine (DWX-52DCi, manufactured by DGSHAPE Corporation) according to the manual, and the dimensions ($X_{PS}$, $Y_{PS}$, $Z_{PS}$) were measured using a coolant-proof micrometer (MDC-25MX, manufactured by Mitutoyo Corporation) according to the manual. The zirconia pre-sintered body was then fired at 1450 °C for 2 hours using a dental laboratory porcelain firing furnace (AUSTROMAT 674i, manufactured by DEKEMA Dental-Keramikofen GmbH) according to the manual to obtain a zirconia sintered body. The dimensions of the length, the width, and the height ($X_S$, $Y_S$, $Z_S$) after perfect sintering were measured using a coolant-proof micrometer (MDC-25MX, manufactured by Mitutoyo) according to the manual, and the processing coefficient after pre-sintering at temperature T was calculated using formula 3. Mass measurements were performed using an electronic balance (XP-204, manufactured by Mettler Toledo) according to the manual. After plotting the temperature T on the horizontal axis and the processing coefficient at temperature T on the vertical axis, adjacent plots along the horizontal axis were connected with straight lines. By determining the temperature at which the lines of respective zirconia powders for layering intersect, the pre-sintering temperature was determined so that the processing coefficients of respective layers after pre-sintering are substantially equal.

(Example 1)

Zpex in an amount of 240 g was filled into a $\Phi 100$ mm mold and powder compacted at a surface pressure of 4.78 kN/cm$^2$ for 30 seconds to obtain a first layer. Zpex Smile in an amount of 240 g was filled on the upper surface of the first layer, and powder compacted at a surface pressure of 2.77 kN/cm$^2$ for 60 seconds to obtain a second layer (final layer). The obtained multilayer zirconia green compact was subjected to CIP treatment by pressing at a pressure of 200 MPa for 60 seconds using a cold isostatic press machine. Mass measurements were performed using an electronic balance (XP-204, manufactured by Mettler Toledo) according to the manual. The multilayer zirconia green compact after the CIP treatment was pre-sintered for 30 minutes at 957 °C, which is the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Example 2)

Zpex in an amount of 240 g was filled into a $\Phi$100 mm mold and powder compacted at a surface pressure of 4.78 $kN/cm^2$ for 30 seconds to obtain a first layer. Zpex Smile in an amount of 240 g was filled on the upper surface of the first layer, and powder compacted at a surface pressure of 3.15 $kN/cm^2$ for 60 seconds to obtain a second layer (final layer). The obtained multilayer zirconia green compact was subjected to CIP treatment by pressing at a pressure of 200 MPa for 60 seconds using a cold isostatic press machine. Mass measurements were performed using an electronic balance (XP-204, manufactured by Mettler Toledo) according to the manual. The multilayer zirconia green compact after the CIP treatment was pre-sintered for 30 minutes at 976 °C, which is the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Example 3)

Zpex in an amount of 240 g was filled into a $\Phi$100 mm mold and powder compacted at a surface pressure of 4.78 $kN/cm^2$ for 30 seconds to obtain a first layer. Zpex Smile in an amount of 240 g was filled on the upper surface of the first layer, and powder compacted at a surface pressure of 3.97 $kN/cm^2$ for 60 seconds to obtain a second layer (final layer). The obtained multilayer zirconia green compact was subjected to CIP treatment by pressing at a pressure of 200 MPa for 60 seconds using a cold isostatic press machine. Mass measurements were performed using an electronic balance (XP-204, manufactured by Mettler Toledo) according to the manual. The multilayer zirconia green compact after the CIP treatment was pre-sintered for 30 minutes at 984 °C, which is the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Example 4)

Zpex in an amount of 240 g was filled into a $\Phi$100 mm mold and powder compacted at a surface pressure of 3.15 $kN/cm^2$ for 30 seconds to obtain a first layer. Zpex Smile in an amount of 240 g was filled on the upper surface of the first layer, and powder compacted at a surface pressure of 2.04 $kN/cm^2$ for 60 seconds to obtain a second layer (final layer). The obtained multilayer zirconia green compact was subjected to CIP treatment by pressing at a pressure of 200 MPa for 60 seconds using a cold isostatic press machine. Mass measurements were performed using an electronic balance (XP-204, manufactured by Mettler Toledo) according to the manual. The multilayer zirconia green compact after the CIP treatment was pre-sintered for 30 minutes at 955 °C, which is the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Example 5)

Zpex in an amount of 160 g was filled into a $\Phi$100 mm mold and powder compacted at a surface pressure of 4.78 $kN/cm^2$ for 30 seconds to obtain a first layer. Zpex4 in an amount of 160 g was filled on the upper surface of the first layer, and powder compacted at a surface pressure of 3.01 $kN/cm^2$ for 30 seconds to obtain a second layer. Zpex Smile in an amount of 160 g was filled on the upper surface of the second layer, and powder compacted at a surface pressure of 2.77 $kN/cm^2$ for 30 seconds to obtain a third layer (final layer). The obtained multilayer zirconia green compact was subjected to CIP treatment by pressing at a pressure of 200 MPa for 60 seconds using a cold isostatic press machine. Mass measurements were performed using an electronic balance (XP-204, manufactured by Mettler Toledo) according to the manual. The multilayer zirconia green compact after the CIP treatment was pre-sintered for 30 minutes at 960 °C, which is the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Example 6)

Zpex in an amount of 240 g was filled into a $\Phi$100 mm mold and powder compacted at a surface pressure of 4.78 kN/cm$^2$ for 30 seconds to obtain a first layer. Zpex Smile in an amount of 240 g was filled on the upper surface of the first layer, and powder compacted at a surface pressure of 2.77 kN/cm$^2$ for 60 seconds to obtain a second layer (final layer). The obtained multilayer zirconia green compact was subjected to WIP treatment by pressing at a pressure of 200 MPa and 90 °C for 60 seconds using a warm isostatic press machine. Mass measurements were performed using an electronic balance (XP-204, manufactured by Mettler Toledo) according to the manual. The multilayer zirconia green compact after the CIP treatment was pre-sintered for 30 minutes at 942 °C, which is the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Example 7)

Zpex in an amount of 240 g was filled into a $\Phi$100 mm mold and powder compacted at a surface pressure of 4.78 kN/cm$^2$ for 30 seconds to obtain a first layer. Zpex Smile in an amount of 240 g was filled on the upper surface of the first layer, and powder compacted at a surface pressure of 2.77 kN/cm$^2$ for 60 seconds to obtain a second layer (final layer). The obtained multilayer zirconia green compact was subjected to CIP treatment by pressing at a pressure of 50 MPa for 60 seconds using a cold isostatic press machine. Mass measurements were performed using an electronic balance (XP-204, manufactured by Mettler Toledo) according to the manual. The multilayer zirconia green compact after the CIP treatment was pre-sintered for 30 minutes at 957 °C, which is the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Example 8)

Zpex in an amount of 240 g was filled into a $\Phi$100 mm mold and powder compacted at a surface pressure of 4.78 kN/cm$^2$ for 30 seconds to obtain a first layer. Zpex Smile in an amount of 240 g was filled on the upper surface of the first layer, and powder compacted at a surface pressure of 2.77 kN/cm$^2$ for 60 seconds to obtain a second layer (final layer). The obtained multilayer zirconia green compact was subjected to CIP treatment by pressing at a pressure of 200 MPa for 10 seconds using a cold isostatic press machine. Mass measurements were performed using an electronic balance (XP-204, manufactured by Mettler Toledo) according to the manual. The multilayer zirconia green compact after the CIP treatment was pre-sintered for 30 minutes at 957 °C, which is the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Example 9)

Zpex in an amount of 240 g was filled into a $\Phi$100 mm mold and powder compacted at a surface pressure of 4.78 kN/cm$^2$ for 30 seconds to obtain a first layer. Zpex Smile in an amount of 240 g was filled on the upper surface of the first layer, and powder compacted at a surface pressure of 2.77 kN/cm$^2$ for 10 seconds to obtain a second layer (final layer). The obtained multilayer zirconia green compact was subjected to CIP treatment by pressing at a pressure of 200 MPa for 60 seconds using a cold isostatic press machine. Mass measurements were performed using an electronic balance (XP-204, manufactured by Mettler Toledo) according to the manual. The multilayer zirconia green compact after the CIP treatment was pre-sintered for 30 minutes at 957 °C, which is the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Reference Example 1)

Zpex in an amount of 240 g was filled into a $\Phi$100 mm mold and powder compacted at a surface pressure of 4.78 kN/cm$^2$ for 30 seconds to obtain a first layer. Zpex in an amount of 240 g was filled on the upper surface of the first layer, and powder compacted at a surface pressure of 2.77 kN/cm$^2$ for 60 seconds to obtain a second layer (final layer). The obtained multilayer zirconia green compact was subjected to CIP treatment by pressing at a pressure of 200 MPa for 60 seconds using a cold isostatic press machine. Mass measurements were performed using an electronic balance (XP-204, manufactured by Mettler Toledo) according to the manual. The multilayer zirconia green compact after the CIP treatment was pre-sintered for 30 minutes at 957 °C, which is the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Comparative Example 1)

Zpex in an amount of 240 g was filled into a $\Phi$100 mm mold and powder compacted at a surface pressure of 4.78 $kN/cm^2$ for 30 seconds to obtain a first layer. Zpex Smile in an amount of 240 g was filled on the upper surface of the first layer, and powder compacted at a surface pressure of 4.78 $kN/cm^2$ for 60 seconds to obtain a second layer (final layer). The obtained multilayer zirconia green compact was subjected to CIP treatment by pressing at a pressure of 200 MPa for 60 seconds using a cold isostatic press machine. Mass measurements were performed using an electronic balance (XP-204, manufactured by Mettler Toledo) according to the manual. The multilayer zirconia green compact after the CIP treatment was pre-sintered for 30 minutes at 981 °C, which is the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Comparative Example 2)

Zpex in an amount of 240 g was filled into a $\Phi$100 mm mold and smoothed with a plastic spatula to obtain a first layer. Zpex Smile in an amount of 240 g was filled on the upper surface of the first layer, and powder compacted at a surface pressure of 4.78 $kN/cm^2$ for 60 seconds to obtain a second layer (final layer). The obtained multilayer zirconia green compact was subjected to CIP treatment by pressing at a pressure of 200 MPa for 60 seconds using a cold isostatic press machine. Mass measurements were performed using an electronic balance (XP-204, manufactured by Mettler Toledo) according to the manual. The multilayer zirconia green compact after the CIP treatment was pre-sintered for 30 minutes at 981 °C, which is the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Comparative Example 3)

Zpex in an amount of 240 g was filled into a $\Phi$100 mm mold and powder compacted at a surface pressure of 4.78 $kN/cm^2$ for 30 seconds to obtain a first layer. Zpex Smile in an amount of 240 g was filled on the upper surface of the first layer, and powder compacted at a surface pressure of 2.77 $kN/cm^2$ for 60 seconds to obtain a second layer (final layer). The obtained multilayer zirconia green compact was subjected to CIP treatment by pressing at a pressure of 200 MPa for 60 seconds using a cold isostatic press machine. Mass measurements were performed using an electronic balance (XP-204, manufactured by Mettler Toledo) according to the manual. The multilayer zirconia green compact after the CIP treatment was pre-sintered for 30 minutes at 1000 °C, which is not the temperature specified in the above section [Specifying pre-sintering temperature so that the processing coefficients of each layer after pre-sintering are substantially equal], using a dental laboratory porcelain furnace (AUSTROMAT 674i, manufactured by DEKEMA Dental-Keramikofen GmbH) according to the manual, to obtain a dental zirconia pre-sintered body for cutting and machining.

(Comparative Example 4)

Zpex in an amount of 240 g was filled into a $\Phi$100 mm mold and powder compacted at a surface pressure of 4.78 $kN/cm^2$ for 30 seconds to obtain a first layer. Zpex Smile in an amount of 240 g was filled on the upper surface of the first layer, and powder compacted at a surface pressure of 2.77 $kN/cm^2$ for 60 seconds to obtain a second layer (final layer) to use as the multilayer zirconia green compact without performing isostatic press process. Mass measurements were performed using an electronic balance (XP-204, manufactured by Mettler Toledo) according to the manual. It was impossible to identify pre-sintering temperature so that the processing coefficients of each layer after pre-sintering are substantially equal by the above section [Specifying pre-sintering temperature so that the processing coefficients of each layer after pre-sintering are substantially equal]. Therefore, the obtained multilayer zirconia green compact was pre-sintered for 30 minutes at the pre-sintering temperature (957 °C) that was the same as in Example 1, using a dental laboratory porcelain furnace (AUSTROMAT 674i, manufactured by DEKEMA Dental-Keramikofen GmbH) according to the manual, to obtain a dental zirconia pre-sintered body for cutting and machining.

[Measurement of warpage of pre-sintered body]

Warpage amount was measured for the dental zirconia pre-sintered body for cutting and machining prepared in each of Examples and Comparative Examples using a Digimatic Indicator (ID-SX2, Mitutoyo Corporation) as described above in [Warpage amount of dental zirconia pre-sintered body for cutting and machining]. The measurement results are shown in Table 2.

[Maximum difference in processing coefficient between respective layers]

The maximum difference in processing coefficient between respective layers of the dental zirconia pre-sintered body for cutting and machining prepared in Examples and Comparative Examples was measured using a coolant-proof micrometer (MDC-25MX, manufactured by Mitutoyo Corporation) as described in the above [Maximum difference in processing coefficients]. The measurement results are shown in Table 2.

The results of measuring the amount of warpage of the dental zirconia pre-sintered bodies for cutting and machining of Examples 1 to 9, Reference Example 1, and Comparative Examples 1 to 4 are shown in Table 2. The warpage amount of 0.5 mm or less was evaluated as "warpage not causing a problem in use" and the warpage amount of more than 0.5 mm was evaluated as "warpage causing a problem in use".

[Tabel 2]

| | Example 1 | | Example 2 | | Example 3 | | Example 4 | | Example 5 | | | Example 6 | |
|---|---|---|---|---|---|---|---|---|---|---|---|---|---|
| Layer | 1 | 2 | 1 | 2 | 1 | 2 | 1 | 2 | 1 | 2 | 3 | 1 | 2 |
| Zirconia powder | Zpex | Zpex Smile | Zpex | Zpex Smile | Zpex | Zpex Smile | Zpex | Zpex Smile | Zpex | Zpex4 | Zpex Smile | Zpex | Zpex Smile |
| Relative density of green compact after pressing at 4.78kN/cm² | 46.95 | 49.13 | 46.95 | 49.13 | 46.95 | 49.13 | 46.95 | 49.13 | 46.95 | 48.52 | 49.13 | 46.95 | 49.13 |
| Type of zirconia powder | First | Second | First | Second | First | Second | First | Second | First | Second | Third | First | Second |
| Powder compacting pressure for each layer (kN/cm³) | 4.78 | 2.77 | 4.78 | 3.15 | 4.78 | 3.97 | 3.15 | 2.04 | 4.78 | 3.01 | 2.77 | 4.78 | 2.77 |
| Holding time of press presure in powder compacting press (sec) | 30 | 60 | 30 | 60 | 30 | 60 | 30 | 60 | 30 | 30 | 60 | 30 | 60 |
| Relative density of each layer after powder compacting press | 46.95 | 4703 | 46.95 | 47.64 | 46.95 | 48.51 | 45.46 | 45.65 | 46.95 | 47.02 | 47.03 | 46.95 | 47.03 |
| Relative density difference between green compacts constituting respective layers | 0.08 | | 0.69 | | 1.56 | | 0.19 | | 0.08 | | | 0.08 | |
| Isostatic pressing pressure (MPa) | CIP :200 | | CIP :200 | | CIP :200 | | CIP :200 | | CIP :200 | | | WIP :200 | |
| Holding time of press presure press treatment (sec) | 60 | | 60 | | 60 | | 60 | | 60 | | | 60 | |
| in isostatic temperature (°C) | 957 | | 976 | | 984 | | 955 | | 960 | | | 942 | |
| Warpage of pre-sintered body (mm) | 0.04 | | 0.24 | | 0.37 | | 0.15 | | 0.11 | | | 0.06 | |
| Maximum difference in processing coefficient between respective layers | 0.001 | | 0.001 | | 0.001 | | 0.001 | | 0.001 | | | 0.001 | |

| | Example 7 | | Example 8 | | Example 9 | | Reference Example 1 | |
|---|---|---|---|---|---|---|---|---|
| Layer | 1 | 2 | 1 | 2 | 1 | 2 | 1 | 2 |
| Zirconia powder | Zpex | Zpex Smile | Zpex | Zpex Smile | Zpex | Zpex Smile | Zpex Smile | Zoex Smile |
| Relative density of green compact after pressing at 4.78kN/cm$^2$ | 46.95 | 49.13 | 46.95 | 49.13 | 46.95 | 49.13 | 49.13 | 49.13 |
| Type of zirconia powder | First | Second | First | Second | First | Second | First | Second |
| Powder compacting pressure for each layer (kN/cm$^3$) | 4.78 | 2.77 | 4.78 | 2.77 | 4.78 | 2.77 | 2.77 | 2.77 |
| Holding time of press presure in powder compacting press (sec) | 30 | 60 | 30 | 60 | 30 | 10 | 30 | 60 |
| Relative density of each layer after powder compacting press | 46.95 | 4703 | 46.95 | 47.03 | 46.95 | 47.03 | 47.03 | 47.03 |
| Relative density difference between green compacts | 0.08 | | 0.08 | | 0.08 | | 0.00 | |
| constituting respective layers Isostatic pressing pressure (MPa) | CIP :50 | | CIP :200 | | CIP :200 | | CIP :200 | |
| Holding time of press presure press treatment (sec) in isostatic | 60 | | 10 | | 60 | | 60 | |
| press temperature (°C) | 957 | | 957 | | 957 | | 957 | |
| Warpage of pre-sintered body (mm) | 0.39 | | 0.28 | | 0.15 | | 0.00 | |
| Maximum difference in processing coefficient between respective layers | 0.002 | | 0.002 | | 0.001 | | 0.000 | |

| | Comparative Example 1 | | Comparative Example 2 | | Comparative Example 3 | | Comparative Example 4 | |
|---|---|---|---|---|---|---|---|---|
| Layer | 1 | 2 | 1 | 2 | 1 | 2 | 1 | 2 |
| Zirconia powder | Zpex | Smile | Zpex | Smile | Zpex | Zoex Smile | Zpex | Smile |
| Relative density of green compact after pressing at 4.78kN/cm$^2$ | 46.95 | 49.13 | 46.95 | 49.13 | 46.95 | 49.13 | 46.95 | 49.13 |
| Type of zirconia powder | First | Second | First | Second | First | Second | First | Second |
| Powder compacting pressure for each layer (kN/cm$^3$) | 4.78 | 4.78 | - | 4.78 | 4.78 | 2.77 | 4.78 | 2.77 |
| Holding time of press presure in powder compacting press (sec) | 30 | 60 | - | 60 | 30 | 60 | 30 | 60 |

(continued)

|  | Comparative Example 1 | | Comparative Example 2 | | Comparative Example 3 | | Comparative Example 4 | |
|---|---|---|---|---|---|---|---|---|
| Relative density of each layer after powder compacting press | 46.95 | 49.13 | - | 49.13 | 46.95 | 47.03 | 46.95 | 47.03 |
| Relative density difference between green compacts constituting respective layers | 2.18 | | - | | 0.08 | | 0.08 | |
| Isostatic pressing pressure (MPa) | CIP :200 | | CIP :200 | | CIP :200 | | - | |
| Holding time of press pressure in isostatic press treatment (sec) | 60 | | 60 | | 60 | | - | |
| Pre-sintering temperature (°C) | 981 | | 981 | | 1000 | | 957 | |
| Warpage of pre-sintered body (mm) | 0.56 | | 0.58 | | 0.53 | | 0.54 | |
| Maximum difference in processing coefficient between respective layers | 0.001 | | 0.001 | | 0.004 | | 0.009 | |

In Examples 1 to 9 and Reference Example 1, the warpage amount of the dental zirconia pre-sintered body for cutting and machining was 0.5 mm or less. Further, it was confirmed that the smaller the difference in relative density between respective layers after respective press, the smaller the warpage amount.

In Comparative Example 1, the difference in the relative density between the layers was not adjusted. After powder compacting, the difference in the relative density between the layers was 2.18, and the warpage of the zirconia pre-sintered body exceeded 0.5 mm.

In Comparative Example 2, the powder compacting was not performed on the first layer. In this case, the first layer was pressed with the same press pressure as that in the press of the second layer. Therefore, the relative densities of the respective layers were not adjusted and the warpage of the zirconia pre-sintered body exceeded 0.5 mm.

In Comparative Example 3, the pre-sintering was performed at a temperature other than the temperature at which the processing coefficients were substantially equal. The zirconia pre-sintered body prepared under these conditions had a difference in the processing coefficient between the layers exceeding 0.003 and the warpage of the zirconia pre-sintered body exceeded 0.5 mm.

In Comparative Example 4, the multilayer zirconia green compact was not subjected to isostatic pressing process. In this case, a pre-sintering temperature at which the processing coefficients were substantially equal could not be calculated, so pre-sintering was performed at the pre-sintering temperature used in Example 1. In this case, the warpage of the zirconia pre-sintered body exceeded 0.5 mm.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

Industrial applicability

The present invention can manufacture a dental zirconia pre-sintered body for cutting and machining with little warpage without limiting the powder composition to be stacked to specific coloring materials or stabilizers, and with no distortion after perfect sintering.

**Claims**

1.  A method of manufacturing a zirconia pre-sintered body for dental cutting and machining which has a multilayer structure in which two or more layers are stacked, comprising steps of:

    forming a green compact that constitutes a first layer by filling a mold with a zirconia powder for stacking and by powder compacting,
    forming a multilayer zirconia green compact by forming a second or more layers by filling a position adjacent the formed green compact in the mold with a zirconia powder for stacking and by powder compacting,
    performing isostatic press process on multilayer zirconia green compact, and
    pre-sintering the multilayer zirconia green compact performed with the isostatic press process, wherein
    in the step of forming a second or more layers,

    a zirconia powder for layering used in a green compact constituting each layer is different from a zirconia powder for layering used in a green compact constituting adjacent layer in relative density to the perfect sintered body in the green compact pressed at a press pressure of 4.78 kN/cm$^2$, and
    each press pressure of the powder compacting in forming the green compact that constitutes each layer is adjusted so that the difference in relative density between the green compacts constituting respective layers is 2 or less, and

    in the step of pre-sintering,
    the multilayer zirconia green compact is pre-sintered at a temperature at which the processing coefficients of respective layers after pre-sintering are substantially equal.

2.  The method of manufacturing a zirconia pre-sintered body for dental cutting and machining according to claim 1, wherein
    in the step of forming a multilayer zirconia green compact,
    each press pressure of the powder compacting in forming the green compact that constitutes each layer is adjusted so that the difference in relative density between the green compacts constituting each layer is 1 or less.

3.  The method of manufacturing a zirconia pre-sintered body for dental cutting and machining according to claim 1, wherein
    in the step of forming a multilayer zirconia green compact,
    each press pressure of the powder compacting in forming the green compact that constitutes each layer is adjusted so that the difference in relative density between the green compacts constituting each layer is 0.5 or less.

4.  The method of manufacturing a zirconia pre-sintered body for dental cutting and machining according to any one of claims 1 to 3, wherein

    when the relative densities of the green compacts obtained by pressing the zirconia powder for layering used in the green compacts constituting the first to $n^{th}$ layers at a pressure of 4.78 kN/cm$^2$ are defined as $D_1$ to $D_n$, respectively,
    the relationship $D_1 < D_2 < ... < D_{n-1} < D_n$ is satisfied.

5.  The method of manufacturing a zirconia pre-sintered body for dental cutting and machining according to any one of claims 1 to 4, wherein

    when the press pressures in forming the green compacts constituting the first layer to $n^{th}$ layer are defined as $P_1$ to $P_n$, respectively,
    the relationship the relationship $P_n < P_{n-1} < ... P_2 < P_1$ is satisfied.

6.  The method of manufacturing a zirconia pre-sintered body for dental cutting and machining according to any one of claims 1 to 5, wherein
    the zirconia powder for layering contains a sintering aid.

7.  The method of manufacturing a zirconia pre-sintered body for dental cutting and machining according to any one of claims 1 to 6, wherein
    the zirconia powder for layering contains 80 % by weight of zirconia in terms of weight.

8.  The method of manufacturing a zirconia pre-sintered body for dental cutting and machining according to any one of claims 1 to 7, wherein
    a holding time of the press pressure is 1 second or more in forming the green compact constituting each layer.

9.  The method of manufacturing a zirconia pre-sintered body for dental cutting and machining according to any one of claims 1 to 8, wherein
    in the step of performing isostatic press process, cold isostatic pressing (CIP) and/or warm isostatic pressing (WIP) are performed.

10. The method of manufacturing a zirconia pre-sintered body for dental cutting and machining according to claim 9, wherein
    the pressure of the cold isostatic pressing (CIP) and/or warm isostatic pressing (WIP) is 100 MPa or more.

11. The method of manufacturing a zirconia pre-sintered body for dental cutting and machining according to claim 10, wherein
    the holding time of the press presure of the maximum pressure in cold isostatic pressing (CIP) and/or warm isostatic pressing (WIP) is 30 seconds or more.

12. The method of manufacturing a zirconia pre-sintered body for dental cutting and machining according to any one of claims 1 to 11, wherein
    the multilayer zirconia green compact performed with the isostatic press process is pre-sintered at 800 to 1200 °C.

13. A zirconia pre-sintered body for dental cutting and machining manufactured by the method of manufacturing a zirconia pre-sintered body for dental cutting and machining according to any one of claims 1 to 12.

Europäisches
Patentamt

European
Patent Office

Office européen
des brevets

# EUROPEAN SEARCH REPORT

**Application Number**

EP 25 18 0396

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/327319 A1 (LI HONGWEN [CN] ET AL) 15 November 2018 (2018-11-15) * paragraphs [0039] - [0060] * * the whole document * ----- | 1-13 | INV. A61C13/00 |
| X | US 2022/017423 A1 (KATO SHINICHIRO [JP] ET AL) 20 January 2022 (2022-01-20) * paragraphs [0056] - [0077] * * the whole document * ----- | 1-13 | |
| X | US 2023/338123 A1 (JAHNS MICHAEL [DE] ET AL) 26 October 2023 (2023-10-26) * the whole document * ----- | 1-13 | |

| TECHNICAL FIELDS SEARCHED (IPC) |
|---|
| A61C |

The present search report has been drawn up for all claims

| Place of search | Date of completion of the search | Examiner |
|---|---|---|
| Munich | 7 November 2025 | Salvatore, Claudio |

EPO FORM 1503 03.82 (P04C01)

## ANNEX TO THE EUROPEAN SEARCH REPORT
## ON EUROPEAN PATENT APPLICATION NO.

EP 25 18 0396

This annex lists the patent family members relating to the patent documents cited in the above-mentioned European search report.
The members are as contained in the European Patent Office EDP file on
The European Patent Office is in no way liable for these particulars which are merely given for the purpose of information.

07-11-2025

| Patent document cited in search report | | Publication date | Patent family member(s) | | Publication date |
|---|---|---|---|---|---|
| US 2018327319 | A1 | 15-11-2018 | CN | 107175747 A | 19-09-2017 |
| | | | EP | 3400929 A1 | 14-11-2018 |
| | | | US | 2018327319 A1 | 15-11-2018 |
| US 2022017423 | A1 | 20-01-2022 | CN | 113194905 A | 30-07-2021 |
| | | | EP | 3903761 A1 | 03-11-2021 |
| | | | JP | 7213268 B2 | 26-01-2023 |
| | | | JP | WO2020138316 A1 | 14-10-2021 |
| | | | KR | 20210099119 A | 11-08-2021 |
| | | | US | 2022017423 A1 | 20-01-2022 |
| | | | WO | 2020138316 A1 | 02-07-2020 |
| US 2023338123 | A1 | 26-10-2023 | CN | 116457200 A | 18-07-2023 |
| | | | EP | 4225579 A1 | 16-08-2023 |
| | | | JP | 2023546824 A | 08-11-2023 |
| | | | US | 2023338123 A1 | 26-10-2023 |
| | | | WO | 2022074494 A1 | 14-04-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020147494 A
- JP 6393328 B